# EUROPEAN PATENT SPECIFICATION

(11) **EP 0 527 758 B1**
(45) Date of publication and mention of the grant of the patent: **07.01.1998**
(21) Application number: 91906996.3
(22) Date of filing: 08.04.1991
(51) Int. Cl.: C12N 15/53, C12N 9/04

(54) **RECOMBINANT YEASTS CONTAINING THE DNA SEQUENCES CODING FOR XYLOSE REDUCTASE AND XYLITOL DEHYDROGENASE ENZYMES**
MIT DEN FÜR XYLOSEREDUKTASE UND XYLITOLDEHYDROGENASE KODIERENDEN DNS-SEQUENZEN REKOMBINIERTE HEFEN
LEVURES RECOMBINANTES CONTENANT LES SEQUENCES D'A.D.N. CODANT POUR LES ENZYMES XYLOSE REDUCTASE ET XYLITOL DEHYDROGENASE

(30) Priority: 06.04.1990 FI 901771
(43) Date of publication of application: 24.02.1993
(73) Proprietor: XYROFIN OY, 00240 Helsinki (FI)
(72) Inventor: HALLBORN, Johan, S-222 34 Lund (SE); PENTTILÄ, Merja, SF-00390 Helsinki (FI); OJAMO, Heikki, SF-02600 Espoo (FI); WALFRIDSSON, Mats, S-222 34 Lund (SE); Airaksinen, Ulla, SF-01300 Vantaa (FI); KERÄNEN, Sirkka, SF-00240 Helsinki (FI); HAHN-HÄGERDAL, Bärbel, S-223 61 Lund (SE)
(74) Representative: Woods, Geoffrey Corlett
(86) International application number: FI9100103
(87) International publication number: WO9115588

(56) References cited:
- Curr Genet, Vol. 18, September 1990, PETER KOETTER et al.: "Isolation and characterization of the Pichia stipitis xylitol dehydrogenase gene, XYL2, and construction of a xylose-utilizing Saccharomyces cerevisiae transformant", see page 493 - page 500.
- Appl. Biochemistry and Biotechnology, Vol. 26, No. 2, 1990, VINA W. YANG et al.: "Purification and Properties of Xylitol Dehydrogenase from the Xylose- Fermenting Yeast Candida shehatae", see page 197 - page 206, specially the Abstract and the discussion.
- Journal of Fermentations and Bioengineering, Vol. 67, No. 1, 1989, MANFRED RIZZI et al.: "Purification and Properties of the NAD-Xylitol-Dehydrogenase from the Yeast Pichia stipitis", see page 20 - page 24, see the Abstract.
- Curr Genet, Vol. 16, 1989, JUTTA HAGEDORN and MICHAEL CIRIACY: "Isolation and characterization of xyl mutants in a xylose-utilizing yeast, Pichia stipitis", see page 27 - page 33, see specially page 32, column 2.
- Process Biochemistry, 1989, BERNARD ALEXANDER PRIOR et al.: "Fermentation of D- xylose by the Yeasts Candida shehatae and Pichia Stipitis Prospects and Problems", see page 21 - page 32, see specially page 24.
- Enzyme Microb. Technol., Vol. 12, January 1990, N.W.Y. HO et al.: "Purification, characterization, and amino: terminal sequence of xylose reductase from Candida shehatae", see page 33 - page 39, see especially pages 35-36, Table 2 page 37, Fig. 5 page 38.
- Appl. Microbiol. Biotechnol., Vol. 29, 1988, MANFRED RIZZI et al.: "Xylose fermentation by yeasts", see page 148 - page 154, see especially discussion page 153, column 2.

## Description

### Field of the invention

This invention relates to recombinant-DNA-technology. Specifically this invention relates to new recombinant yeast strains transformed with xylose reductase and optionally with xylitol dehydrogenase enzyme genes. A yeast strain transformed with the xylose reductase gene is capable of reducing xylose to xylitol and consequently of producing xylitol *in vivo.*

Further, the said new yeast strains are capable of expressing the said two enzymes. Xylose reductase produced by these strains can be used in an enzymatic process for the production of xylitol *in vitro.*

### Background of the invention

### Xylose utilization

Xylose appears in great abundance in nature. It can constitute as much as 40 % of a lignocellulosic material (Ladisch *et al.,* 1983). By fermentation xylose can be converted to ethanol which can be used as a liquid fuel or a chemical feedstock. Enzymatically or as a by-product of fermentation xylose can also be converted to xylitol which is a promising natural sweetener having dental caries reducing properties. Xylitol can also be used by diabetics. For the production of xylitol which is meant for human consumption it is important that the process involves GRAS organisms.

Natural xylose utilizers are found among bacteria, yeast and fungi. In all organisms xylose is converted to xylulose which is phosphorylated to xylulose-5-phosphate (X5P) with xylulokinase. X5P then enters the Embden-Meyerhof pathway (glycolysis) via the pentose phosphate shunt.

Bacteria like *Escherichia coli, Bacillus* sp., *Streptomyces* sp. and *Actinoplanes sp.* convert xylose directly to xylulose with a xylose isomerase (XI). Thus bacteria do not produce xylitol as an intermediate during xylose utilization. Those which ferment xylose to ethanol do so with poor yields because a number of by-products are also produced (Skoog and Hahn-Hägerdal, 1988). In xylose utilizing yeasts such as *Pichia stipitis, Candida shehatae* and *Pachysolen tannophilus* this reaction occurs in two steps: first xylose is reduced to xylitol with a xylose reductase (XR) and the xylitol is oxidized with a xylitol dehydrogenase (XDH) to xylulose.

Pure xylose solutions are fermented with high yields and good productivities by xylose utilizing yeasts such as P. *stipitis, C. shehatae* and P. *tannophilus* (Slininger *et al.,* 1987; Prior *et al.,* 1989). However, they do not generally survive in the hostile environment of an untreated raw material such as eg. spent sulphite liquor or hydrogen fluoride-pretreated and acid-hydrolyzed wheat straw (Lindén and Hahn-Hägerdal, 1989). The one exception, *P. tannophilus*, produces mainly xylitol and glycerol in response to this environment. In order to efficiently ferment such raw materials with the xylose utilizing yeasts such as *P. stipitis, C. shehatae* and *P. tannophilus* the raw material has to undergo expensive pretreatments with ion-exchange resins (Clark and Mackie, 1984) or steam stripping (Yu *et al.,* 1987).

*Saccharomyces cerevisiae,* baker's yeast, ferments spent sulphite liquor or hydrogen fluoride-pretreated and acid-hydrolyzed wheat straw to ethanol (Lindén and Hahn-Hägerdal, 1989). *S. cerevisiae* cannot utilize xylose efficiently and cannot grow on xylose as a sole carbon source. In the presence of the bacterial enzyme xylose isomerase, which converts xylose to xylulose, *S. cerevisiae* can, however, ferment both pure xylose solutions (Hahn-Hägerdal *et al.,* 1986) and untreated raw materials (Lindén and Hahn-Hägerdal, 1989a,b) to ethanol with yields and productivities that are in the same order of magnitude as those obtained in hexose fermentations. Similar results have been obtained with *Schizosaccharomyces pombe* (Lastick *et al.,* 1989). Thus, both S. *cerevisiae* and *Sch. pombe* have a functioning xylulokinase enzyme. It has also been found that *S. cerevisiae* can take up xylose (Batt *et al.,* 1986; van Zyl *et al.,* 1989; Senac and Hahn-Hägerdal, 1990).

Gong (1985) discloses a process for obtaining ethanol directly from D-xylose by xylose fermenting yeast mutants. According to Gong a parent yeast strain is selected (e.g. *Candida* sp. or *Saccharomyces cerevisiae*), which originally may have the ability to utilize D-xylose, and this parent strain is then exposed e.g. to UV-radiation so as to induce mutation. However, no information about the reason why the mutants obtained are able to utilize xylose, is given in the reference. Further, Gong did not introduce any new coding and/or regulatory sequences to said strains by genetic engineering techniques to enhance xylose fermentation.

Xylitol is industrially manufactured at the moment by chemical reduction of hemicellulose hydrolysates. Poisoning of the expensive catalyst used in the reduction step and formation of side-products difficult to be separated from the end product are the main problems in this process.

In literature there are numerous examples of microbiological methods to produce xylitol from pure xylose (eg. Onishi and Suzuki, 1966; Barbosa *et al.,* 1988). Best producers in this method are yeasts especially belonging to the *Candida-genera.* Also some bacteria such as *Enterobacter* (Yoshitake *et al.,* 1973a) and *Corynebacterium* species (Yoshitake *et al.,* 1973b) and some molds eg. *Penicillium chrysogenum* (Chiang and Knight, 1960) produce xylitol from pure xylose.

In a microbiological method describing the best yields of xylitol production (Ojamo *et al.,* 1987) *Candida guilliermondii* yeast is cultivated under strictly controlled aeration in a xylose containing medium either as a batch or a fed-batch process. Xylitol yields 50-65 % were obtained. The yield could be increased to 76 % by adding furfuraldehyde to the cultivation medium.

Cell-free extracts from *Candida pelliculosa* (xylose reductase) and *Methanobacterium* sp. (hydrogenase, F₄₂₀, NADP, F₄₂₀/NADP oxidoreductase) has been used to produce xylitol in a membrane reactor with 90 % conversion (Kitpreechavanich, 1985). With a cell-free extract from a *Corynebacterium* species 69 % conversion has been obtained when 6-phosphogluconate was used for regeneration of the cofactor.

It has been shown that glucose dehydrogenase from *B. megaterium* has suitable properties as a NADPH regenerating enzyme (Kulbe *et al.,* 1987). Thus gluconic acid from glucose can be produced simultaneously with xylitol in the enzymatic process. For the retention of the enzymes and the cofactor one can use ultrafiltration membranes. Cofactor retention may be achieved by the use of a derivatized cofactor having high enough molecular weight for the retention (Kulbe *et al.,* 1987) or better by using negatively charged ultrafiltration membranes (Kulbe *et al.,* 1989).

Attraction to use an enzymatic method is based on the possibility to use impure xylose containing raw materials which in the microbiological methods would inhibit the metabolism of the microbe used. Also the yields of xylitol are higher than in the microbiological methods with natural strains. On the other hand any microbiological method is more simple in large-scale practice at the moment.

The natural xylose utilizing yeasts such as *P. stipitis, Candida* sp. and *P. tannophilus* are not suitable for the production of either ethanol or xylitol for several reasons. The fermentation to ethanol requires pretreatment of the raw material which is cost-prohibitive for a cheap end-product such as ethanol. These species also lack the GRAS-status. Thus xylose utilization would most suitably be based on the use of bakers' yeast which has a GRAS-status.

In order to make *S. cerevisiae* an efficient xylose utilizer for the production of xylitol and ethanol an efficient enzyme system for the conversion of xylose to xylitol and xylulose should be introduced into this yeast. For the production of ethanol from xylose the XI genes from *E. coli* (Sarthy *et al.,* 1987), *B. subtilis* and *Actinoplanes missourienris* (Amore *et al.,* 1989) have been cloned and transformed into *S. cerevisiae.* The XI protein made in *S. cerevisiae* had very low (1/1000 of the enzyme produced in bacteria) or no enzymatic activity. Thus, for some reasons the bacterial enzyme can not be made functional in yeast. Another possibility would be to transfer into *S. cerevisiae* the genes encoding XR and XDH from another yeast. The enzymes of *P. stipitis* should be good candidates in the light of the efficient utilization of pure xylose solutions discussed above. It can be anticipated that enzymes from another yeast would function better than bacterial enzymes when expressed in yeast. In addition, xylitol and ethanol could be produced with the same system and the system would combine the good xylose utilization of *P. stipitis* with resistance to inhibitors and general acceptance of *S. cerevisiae*.

Hagedorn et al (Current Genetics 16: 27-33 (1989)) describes the isolation and characterization of mutants of the xylose-utilizing yeast *Pichia stipitis.* The mutants were deficient in either xylose reductase or xylitol dehydrogenase. The fermentation of xylose to ethanol is stated to be of economical interest.

### Summary of the Invention

According to the present invention, there is provided a process for producing xylitol, which process comprises
a) cultivating in a xylose containing medium a yeast strain transformed with a DNA sequence encoding a xylose reductase enzyme, which DNA sequence when transformed into said yeast strain confers to said yeast strain the ability of reducing xylose to xylitol, and
b) recovering xylitol formed from the medium.

According to the present invention, there is further provided an enzymatic process for producing xylitol, which process comprises
a) cultivating a recombinant yeast strain transformed with a DNA sequence encoding a xylose reductase enzyme under conditions permitting expression of said xylose reductase.
b) recovering said xylose reductase enzyme,
c) using said xylose reductase in an enzyme reactor with a cofactor regenerating system, and
d) isolating and purifying the xylitol produced.

The present application describes the isolation of genes coding for xylose reductase (XR) and xylitol dehydrogenase (XDH) from certain yeasts having these genes, the characterization of the genes and their transfer into, and their expression in *Saccharomyces cerevisiae.*

### Brief description of the drawings

**Fig. 1** shows the xylose reductase gene integrated into pMA91 and pRS305 resulting in plasmids pUA103 and pUA107, respectively.

**Fig. 2** Activity plate assay of a XDH positive λ gt11 clone.

**Fig. 3** shows the picture of the plasmid pJHXDH50 carrying the *xdh* gene.

**Fig. 4** shows a plate activity assay of a recombinant *S. cerevisiae* strain VTT-C-91181 producing XDH, on the original transformation plate (A) and as single colonies obtained from this transformant (B).

**Fig. 5** shows the xylose reductase expression cassette flanked by ribosomal sequences integrated into BS+, generating the vector pJHXR22.

**Fig. 6** shows Western analysis of XR produced in *S. cerevisiae* from the following plasmids and from *P. stipitis*. Lanes: 1 molecular weight standars (LMW, Pharmacia); 2 pRS305; 3 pUA107; 4 empty lane; 5 pMA91; 6 pUA103; 7 purified XR enzyme; 8 LMW; 9 *Pichia stipitis*. Samples were taken from French pressed cell lysates for lanes 2, 3, 5, 6 and 9.

**Fig. 7** shows XR activity of *S. cerevisiae* strains transformed with pUA103 and pUA107 using as a cofactor either NADH or NADPH, and of the control strains carrying the vector pRS305 or pMA91.

**Fig. 8** shows the xylitol dehydrogenase gene integrated into pKB102 generating plasmid pJHXDH60.

### Detailed description of the invention

Xylose reductase (*xrd*) and xylitol dehydrogenase (*xdh*) genes to be used in this invention are isolated from a yeast containing these genes, eg. *Pichia stipitis.* Also other suitable yeasts and other fungi, such as *Pachysolen tannophilus, Kluyveromyces* spp., *Petromyces albertensis* and *Candida* spp. can be used.

The yeast to be transformed with these genes can be any suitable yeast, for xylitol production preferably having the GRAS-status, eg. any *Saccharomyces cerevisiae* yeast strain, (eg. DBY746, AH22, S150-2B, GPY55-15Bα, VTT-A-63015, VTT-A-85068, VTT-C-79093), any *Kluyveromyces* sp. or *Schizosaccharomyces pombe.* Transfer of the genes into these yeasts can be achieved, for instance, by using the conventional methods described for these organisms. It is to be noticed that, if wanted, also *Pichia* itself can be transformed with these genes in order to obtain increased or modulated expression of the genes. *Saccharomyces cerevisiae* strains are preferable for the purposes of this invention.

The DNA sequence coding for XR enzyme is isolated from *P. stipitis* by conventional methods. In the preferred embodiment, cDNA is synthesized from mRNA and cloned into λ gt11 vector. Using immuno screening with XR specific antibodies, the positive clone is isolated and subcloned into BS+ vector for sequencing. Gene encoding XR of *P. stipitis* can be cloned also by expression in *S. cerevisiae* because it does not contain any introns. Another possibility is the use of oligonucleotides, designed on the basis of the amino acid sequence of the enzyme, in hybridization of a gene bank.

To construct a plasmid suitable for transformation into a yeast, the gene coding for XR is cloned into a suitable yeast expression vector, such as pMA91 (Mellor *et al.,* 1983), comprising the appropriate yeast regulatory regions. These regulatory regions can be obtained from yeast genes such as the *PGK1, ADH1, GAL1, GAL10, CUP1, GAP, CYC1, PHO5,* for instance. Alternatively, also the regulatory regions of the *Pichia* gene encoding XR can be used to express the gene in *S. cerevisiae.* The plasmid carrying the xrd gene encoding XR is capable of replicating autonomously when transformed into the recipient yeast strain. The gene encoding XR together with the appropriate yeast regulatory regions can also be cloned into a single copy yeast vector such as pRS305 (Sikorski and Hieter, 1989).

Alternatively, the gene coding for XR can also be integrated into the yeast chromosome, into the ribosomal RNA locus, for instance. For this purpose the ribosomal sequences of a suitable plasmid, eg. plasmid pIRL9 are released, and cloned appropriately to BS+ vector. The gene coding for XR, coupled in between suitable yeast promoter and terminator regions, is released from the hybrid vector comprising the gene and cloned into the plasmid obtained at the previous stage. From this resulting plasmid the expression cassette, flanked by ribosomal sequences can be released. This fragment is cotransformed into a yeast with an autonomously replicating plasmid carrying a suitable marker for transformation. The plasmid can be later on removed from the cells containing the *xrd* gene integrated in the chromosome by cultivating the cells in non-selective conditions. This way, recombinant strains can be obtained which carry no extra foreign DNA such as bacterial vector sequences. If a polyploid yeast strain, such as VTT-A-63015, is used the gene can be integrated also to an essential locus such as the *PGK1* or the *ADH1* locus.

The sequence of the *xrd* gene can be determined, if required, from the plasmids carrying it by using eg. the double stranded dideoxy nucleotide sequencing method (Zagursky *et al.,* 1986). The sequence of the *xrd* gene encoding XR of *P. stipitis* is given as the SEQ ID NO. 2.

Specific yeast vectors comprising the *xrd* gene used to transform yeast strains to be used in the invention may either be an autonomously replicating multicopy or a single copy plasmid or a vector capable of integrating into the yeast chromosome, as described above.

Yeast strains which comprise the DNA sequence coding for XR and are capable of expressing this enzyme may also be used in the process of the invention.

According to a preferred aspect of the invention, a process for producing xylose reductase enzyme is also provided. This process comprises:
(a) isolating the DNA sequence coding for xylose reductase from a suitable donor organism;
(b) constructing a yeast vector carrying said DNA sequence;
(c) transforming the vector obtained into a suitable yeast host to obtain a recombinant host strain;
(d) cultivating said recombinant host strain under conditions permitting expression of said xylose reductase; and
(e) recovering said xylose reductase.

The xylose reductase thus obtained can then be used in an enzyme reactor in the further process according to the invention.

Enzymes from organisms having a GRAS status are preferred in an enzymatic production of xylitol. Xylose reductase from *Pichia stipitis* have excellent enzymatical properties such as kinetic constants and stability without special stabilizing agents such as thiol-protecting chemicals. Production of this enzyme is now possible in a yeast having a GRAS status. The transformed yeast cells are cultivated in an appropriate medium. As the cultivation medium, a cheap medium such as one based on molasses can be used as xylose is not needed for induction as in naturally xylose utilizing yeasts. Yeast with intracellular xylose reductase is produced with a good yield in a fed-batch process.

The yeast is concentrated and washed by eg. centrifugation and xylose reductase is liberated into the solution by cell disruption methods such as high pressure homogenization or glass bead milling. After clarification of the homogenate by filtration or centrifugation xylose reductase is further purified by chromatographic methods.

For the production of xylitol *in vitro* a crude homogenate or purified xylose reductase is used in an enzyme reactor together with a cofactor (NAD/NADH or NADP/NADPH) and a cofactor regenerating enzyme such as glucose dehydrogenase, formate dehydrogenase or any other enzyme having good enough stability, requirements for environmental conditions coping with those of xylose reductase and suitable kinetic properties (Bückmann, 1979; Wandrey *et al,* 1981; 1982; Kulbe *et al.,* 1989). The enzymes and the cofactor are typically kept in the reactor system by ultrafiltration membranes especially those with a negative charge. The cofactors may also be coimmobilized with the cofactor regenerating enzymes (Reslow *et al.,* 1988). The reaction mixture is pumped through the reactor and the substrates and the products are filtered through the membrane. The products can be separated from the substrates by eg. chromatographic or crystallization methods and the substrates can be recycled to the reaction mixture.

Further, this invention provides a microbiological process for producing xylitol which process comprises:
(a) cultivating a recombinant yeast strain carrying a DNA sequence coding for xylose reductase enzyme in xylose containing medium; and
(b) recovering the xylitol formed in the medium.

In a microbiological xylitol production with a recombinant yeast the *in vivo* regeneration of the cofactor NADPH or NADH must be secured in one way or another. With a yeast construction having only xylose reductase and not xylitol dehydrogenase gene, cofactor regeneration can be achieved by adding a co-carbon-substrate such as glucose, glycerol, ribose or ethanol. With such a system, 95-100 % yield of xylitol from xylose can be obtained. In this system with *S. cerevisiae,* xylitol is not metabolized further and consequently higher yields of xylitol can be obtained than with natural xylitol producing organisms. When the yeast has also xylitol dehydrogenase gene (see hereunder) cofactor regeneration may happen through a slight flow of xylitol further in the metabolism. This flow can be controlled by relative amounts of expression of the enzymes xylose reductase and xylitol dehydrogenase or by controlling the metabolism of the yeast by oxygen transfer rate or by adding enzyme inhibitors such as iodoacetate to the cultivation medium.

In the preferred embodiment, for the isolation of the *xdh* gene of *P. stipitis* a chromosomal gene bank is first made into *E. coli* in a cosmid p3030 (Penttilä, *et al.,* 1984) or in another yeast vector, and recombinant plasmids are isolated and transformed into yeast. The *xdh* gene can be found by its expression in yeast, which can be detected by an activity plate assay. A cDNA copy for this gene can be isolated similarly by an activity plate assay from a λ gt11 cDNA expression library made in *E. coli.*

An alternative method for the isolation of the *xdh* gene from *P. stipitis* is to purify XDH from a donor yeast by chromatographic methods and determine the N-terminal amino acid sequence thereof. A mixture of oligonucleotides based on the obtained N-terminal amino acid sequence of XDH protein can be designed. This oligonucleotide mixture can then be used in hybridization of a gene bank, or together with an oligo-dT-primer to amplify by PCR reaction *xdh* specific sequences from a mRNA population. The resulting gene or cDNA is cloned into BS+ vector, or a similar vector, and the sequence of the *xdh* gene is then obtained by conventional methods.

The *xdh* gene can be expressed in yeast from the chromosomal copy cloned into for instance the yeast cosmid p3030 (Penttilä *et al.*, 1984). To such a yeast carrying the *xdh* gene, the plasmid carrying the *xrd* gene can be transformed.

Also, the full length *xdh* cDNA can be cloned into a suitable expression vector, such as pMA91 or pKB102 (Blomqvist *et al.,* 1991) in between appropriate yeast regulatory regions, preferably using the yeast *PGK1* or *ADH1* promoter and terminator. The expression cassette built into pKB102 can be released from the resulting plasmid and cloned into an autonomously replicating yeast multicopy vector or into a single copy yeast vector, which carry a suitable marker, eg. *URA3* or *HIS3* for yeast transformation. These resulting plasmids can then be transformed into a suitable host strain or into the strains carrying the gene encoding XR.

The *xdh* gene can also be integrated into the yeast genome in the same manner as described above for the *xrd* gene.

Yeast strains for use in the invention capable of expressing xylose reductase or coexpressing xylose reductase and xylitol dehydrogenase, may be constructed by a method which comprises:
(a) isolating the DNA sequences coding for xylose reductase and xylitol dehydrogenase from a suitable donor organism;
(b) constructing a yeast vector carrying either of the said DNA sequences; and
(c) transforming the former one of the vectors obtained or both of them into a suitable yeast host.

Active xylose reductase and xylitol dehydrogenase coexpressed in a yeast strain may be used to prepare ethanol, xylitol and acetic acid by a process, which comprises:
(a) isolating the DNA sequences coding for xylose reductase and xylitol dehydrogenase from a suitable donor organism;
(b) constructing yeast vectors each carrying one of said DNA sequences;
(c) transforming the vectors obtained to a suitable host to obtain a recombinant yeast strain;
(d) cultivating said recombinant yeast strain in a xylose containing medium; and
(e) isolating and purifying the products (ethanol, xylitol, acetic acid) formed in the medium.

### Preparative Example 1:

### Purification of xylose reductase from Pichia stipitis

*P. stipitis* was grown in a 1 litre fermentor in xylose containing medium (0.3 % yeast extract, 0.3 % malt extract, 0.5 % peptone, 1.9 % KH₂PO₄, 0.3 % (NH₄)₂HPO₄, 0.1 % MgSO₄ and 5 % xylose, pH 5) and harvested in late logarithmic growth phase. 30 g wet weight of cell paste was disrupted using freeze pressing (X-press) and centrifuged 1500 g, 10 min to get a cell free extract.

The crude extract was concentrated 2-fold and applied to a Sephadex G-200 (Pharmacia, Uppsala, Sweden) column (137 ml). At a flow rate of 6 ml/h the proteins were eluted and fractions (9 ml) containing XR activity were pooled.

The pooled fraction was applied to a DEAE-Sepharose (Pharmacia) column (37 ml) equilibrated with 0.02M ammonium phosphate buffer pH 6.0 and eluted with a 250 ml gradient of 0-05 M NaCl in 0.02 M ammonium phosphate buffer at a flow rate of 12 ml/min. Fractions containing XR activity were pooled (6 ml) and concentrated to 1 ml. 1 ml of the concentrated sample was applied to a 1 ml HPLAC column (cibacron blue F36-A; Perstorp Biolytica, Lund, Sweden) equilibrated with 0.02 M ammonium phosphate buffer pH 6.0. Elution of XR was performed with a 0-2 M NaCl gradient in ammonium phosphate buffer at a flow rate of 1 ml/min. Fractions containing XR activity were pooled (6 ml) and dialysed over night at 4°C.

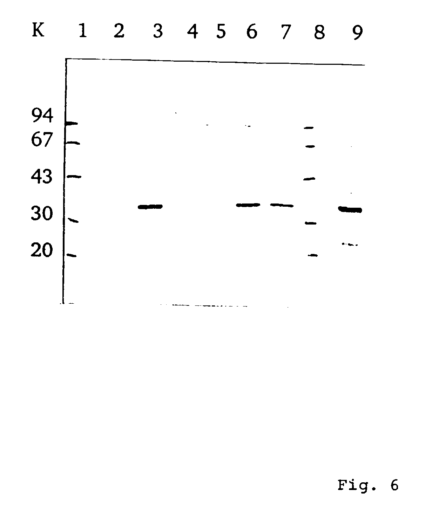

Purity and molecular weight was determined by gradient SDS-polyacrylamide gel (T 8.8-21.3 %, C 2.6 %) electrophoresis (Laemmli, 1970) and native gradient polyacrylamide gel (Pharmacia premade gel, 4/30) electrophoresis (Pharmacia vertical electrophoresis system). Low and high molecular weight standards from Pharmacia were used. Staining of gel was performed with 0.1 % Coomassie Blue R-250 (Sigma) in 25 % methanol and 10 % acetic acid. In SDS-PAGE and in native-PAGE gel the XR fraction after HPLAC appeared as a single band (data not shown). Specific staining of XR with the zymogram technique showed that the single band in the native-PAGE gel was XR (data not shown). Molecular weight estimation with SDS-PAGE (see Fig. 6) and native-PAGE showed a molecular weight of 38000 ± 1000 for the subunits and 76000 ± 1000 for the native protein.

The purified enzyme was used to produce polyclonal antibodies and to make a N-terminal amino acid sequence of the enzyme (Marc Bauman at Dept. of Medical Chemistry, Univ. of Helsinki) (SEQ ID NO. 1).

### Preparative Example 2:

### Cloning of the gene coding for XR from Pichia stipitis

1 litre of *P. stipitis* culture was grown in xylose containing medium (see Preparative example 1) and harvested in late log phase. Harvested cells were converted to sphearoplasts with Zymolyase, suspended into 60 ml of GuSCN solution and RNA was isolated according to Chirgwin *et al.* (1979). RNA was then run through an oligo(dT) cellulose affinity chromatography column. Poly (A+)mRNA was eluted by decreasing the ionic strength of the elution buffer. cDNA was synthesized from mRNA using the cDNA synthesis kit of Amersham and cloned into the λ gt11 vector using the Amersham cDNA cloning kit. After 3-4 h growth, the plaques were replica plated onto nitrocellulose membranes soaked in IPTG, and incubated over night. The membranes were then used for immuno screening of transformants (Young and Davies, 1983) using rabbit antiserum against XR and goat anti rabbit antibodies coupled with alkaline phosphatase. Positive clones were picked from the plates and the insert DNA was amplified with PCR (Güssow and Clackson, 1989) using vector specific primers. The DNA fragments obtained were used for restriction enzyme analysis and for further cloning after BamHI cleavage into BamHI cleaved BS+ (Stratagene) vector. The longest cDNA clone pJHXR20 was sequenced using the double stranded dideoxy nucleotide method (Zagursky *et al.*, 1986).

Verification of the cloning of the full length cDNA coding for XR was obtained by comparing the N-terminal amino acid sequence of the protein with the sequenced gene (SEQ ID NO. 1, SEQ ID NO. 2).

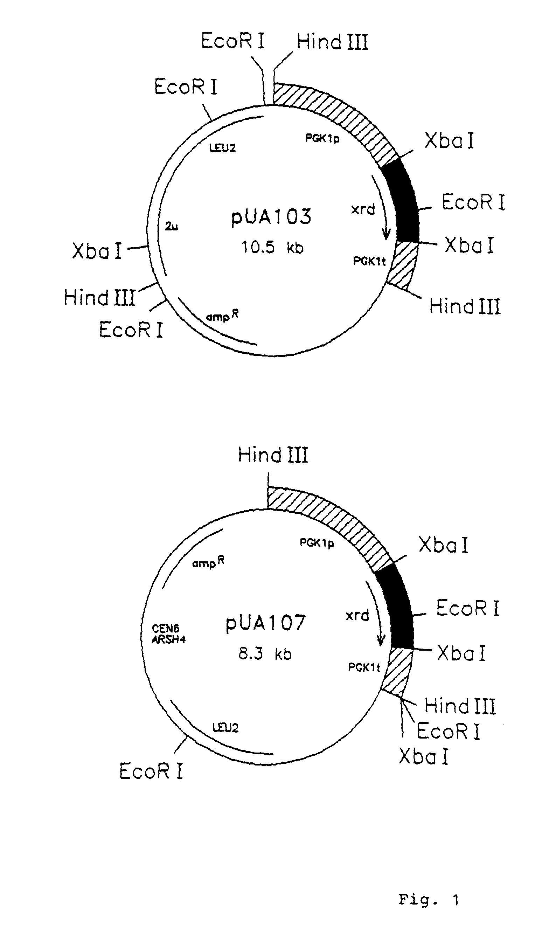

The chromosomal copy of the *xrd* gene was obtained by PCR reaction of total chromosomal DNA isolated (Cryer *et al.,* 1979) from *P. stipitis* CBS-6054 (Prior *et al.,* 1989) using primers corresponding to the 5' (GCGGATCCTCTAGAATGCCTTCTATTAAGTTGAACTCTGG) and to the 3' (TTGGATCCTCTAGATTAGACGAAGATAGGAATCTTGTCCC) end of the coding region and carrying BamHI and XbaI restriction sites. The PCR product was digested with BamHI and cloned into plasmid pMA91 (Mellor *et al.,* 1983) at the BglII site to obtain plasmid pUA103 (Fig. 1). The DNA sequence of the chromosomal copy was compared to that of the cDNA in plasmid pJHXR20, and was shown to contain no introns.

### Preparative Example 3:

### Purification of XDH from Pichia stipitis

*Pichia stipitis* was grown in xylose containing medium as described (Preparative example 1). 30 g wet weight of cell paste was disrupted using freeze pressing (X-press) and centrifuged 1500 g 10 min. The centrifuged cellfree extract was concentrated 3 times and 5 ml was gel filtrated at a flow rate of 6 ml/h through a 137 ml Sepharose 6B column equilibrated with 0.05 M ammonium phosphate buffer pH 6, 25 % glycerol, 1 mM DTT, 1 mM EDTA. Fractions containing XDH activity measured according to Smiley and Bolen (1982) were pooled and applied on a 37 ml ion exchange chromatography column (DEAE-sepharose) equilibrated with 0.05 M ammonium phosphate buffer pH 6, 25 % glycerol, 1 mM DTT, 1 mM EDTA. XDH fractions were eluted with a 250 ml salt gradient of 0-0.5 M NaCl at a flow rate of 12 ml/min and pooled. The partially purified enzyme was run in a polyacrylamide gel and blotted onto a PVD membrane. The band corresponding to XDH was cut out and the N-terminal amino acid sequence was determined directly from the membrane.

### Preparative Example 4:

### Cloning of the gene coding for XDH and expression in S. cerevisiae

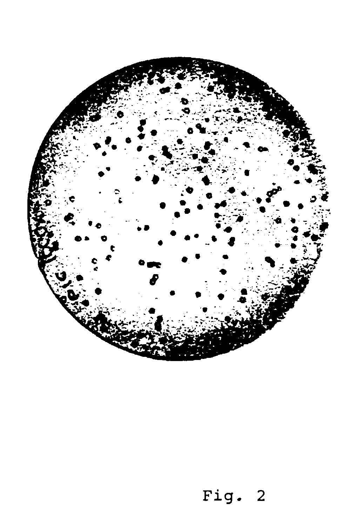

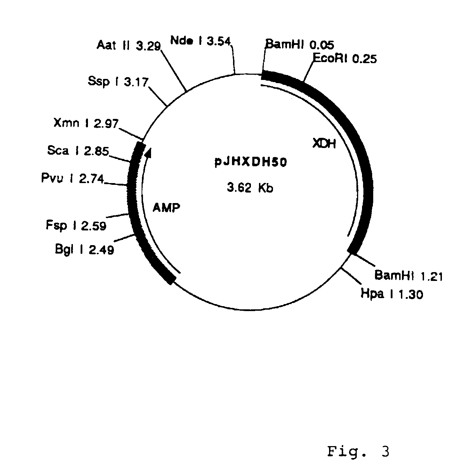

The same λ gt11 cDNA library as obtained and described in Preparative Example 2 was plated and replica plated onto nitrocellulose membrane soaked in IPTG and incubated for 3 hours. The membranes were then used for specific XDH activity (zymogram) screening by soaking the membranes in 10 ml of zymogram solution (0.1 M phosphate buffer pH 7.0, 1.5 mM NAD, 0.25 mM nitroblue tetrazolium, 65 µM phenazinc methosulphate, 0.4 M xylitol). Positive clones (Fig. 2) were picked from the plates and the insert DNA of two of the clones was amplified with PCR (Güssow and Clackson, 1989) using vector specific primers (5'-GGTGGCGACGACTCCTGGAGCCCG, 5'-TTGACACCAGACCAACTGGTAATG). The DNA fragments obtained were of the same size and were used for restriction enzyme analysis to check non-cutting and cutting enzymes and for further cloning after BamHI cleavage into BamHI cleaved pSP72 vector (Promega). The plasmid pJHXDH50 carries the longest cDNA clone (Fig. 3).

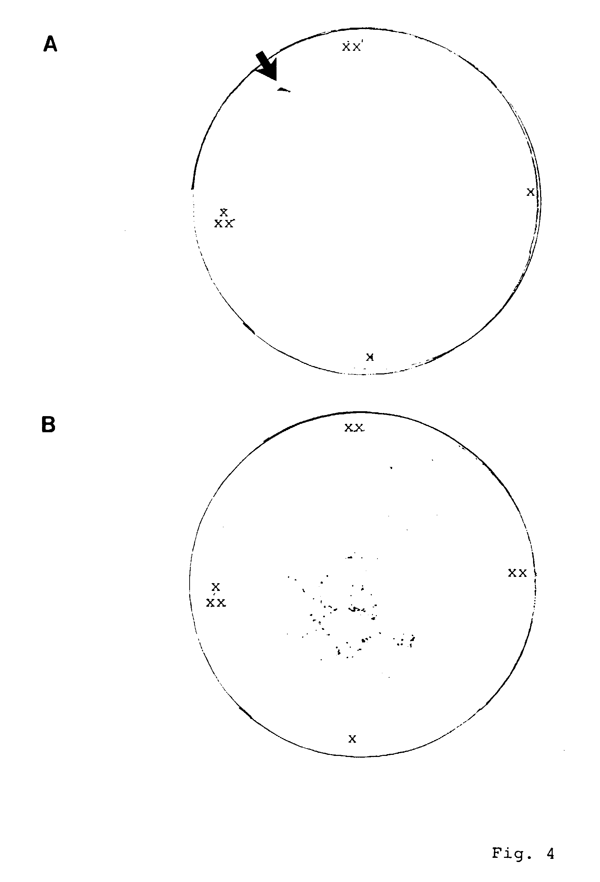

To clone the chromosomal copy of the *xdh* gene, chromosomal DNA was isolated (Cryer *et al.,* 1979) from *P. stipitis* strain CBS-6054 (Prior *et al.,* 1989) and cut partially with Sau 3A. Fragments of 35-45 kb in size were purified by fractionating the partially digested DNA in a sucrose gradient (15 %-40 %), and the fragments were ligated to a p3030 yeast cosmid vector (Penttilä *et al.,* 1984), cut with BamHI. The ligated molecules were packaged into λ particles using the *in vitro* packaging kit of Amersham Ltd. (UK) and transfected into *E. coli* HB101 (Maniatis *et al.,* 1982). Recombinant cosmid DNA was isolated from about 15000 pooled gene bank clones obtained and transformed into a *S. cerevisiae* strain S150-2B (*a, his3-del1, leu2-3, leu2-112, trpl-289, ura3-52,* cir⁺, Gal⁺) (Baldari *et al.,* 1987) by selecting for His⁺ transformants on Sc-his plates. The gene bank was replica plated onto nitrocellulose filters, the cells were broken by incubating the filters for 5 min. in chloroform and the activity assay was performed as described above for the λ gt11 library. Several positive clones were obtained (strains H494, H495, H496, H497 and VTT-C-91181), and the strain VTT-C-91181 (Fig. 4) was used for further studies.

XDH activity of the strain VTT-C-91181 was tested according to Smiley and Bolen (1982) from French pressed total cell lysate containing 25 % glycerol. DNA from VTT-C-91181 was isolated and the plasmid pMW22 rescued by transformation of the DNA into *E. coli* DH5α.

The *S. cerevisiae* strain VTT-C-91181 carrying the plasmid pMW22 was deposited according to the Budapest Treaty with the accession No. NCYC 2352 at the National Collection of Yeast Cultures (NCYC), UK, on March 29, 1991.

### Preparative Example 5:

### Expression of XR in S. cerevisiae

The gene coding for XR with the regulatory regions of the *PGK* gene, was released from the plasmid pUA103 (Preparative Example 2) with HindIII and cloned at the HindIII site of the single copy vector pRS305 (Sikorski and Hieter, 1989). The resulting plasmid pUA107 (Fig. 1), in which the XR encoding gene was in the right orientation towards the yeast *PGK* promoter, was transformed using the LiCl method (Ito *et al.,* 1983), and selecting for Leu⁺ transformants, into *S. cerevisiae* strains S150-2B (see Example 4) and GPY55-15Bα (*leu2-3, leu2-112*, *ura3-52, trpl-289, his4-519, prb1*, cir⁺) giving the new recombinant strains *S*. *cerevisiae* H481 and *S. cerevisiae* H479, respectively. The plasmid pUA103 transformed into the strains S150-2B and GPY-15Ba resulted in strains H477 and H475, respectively.

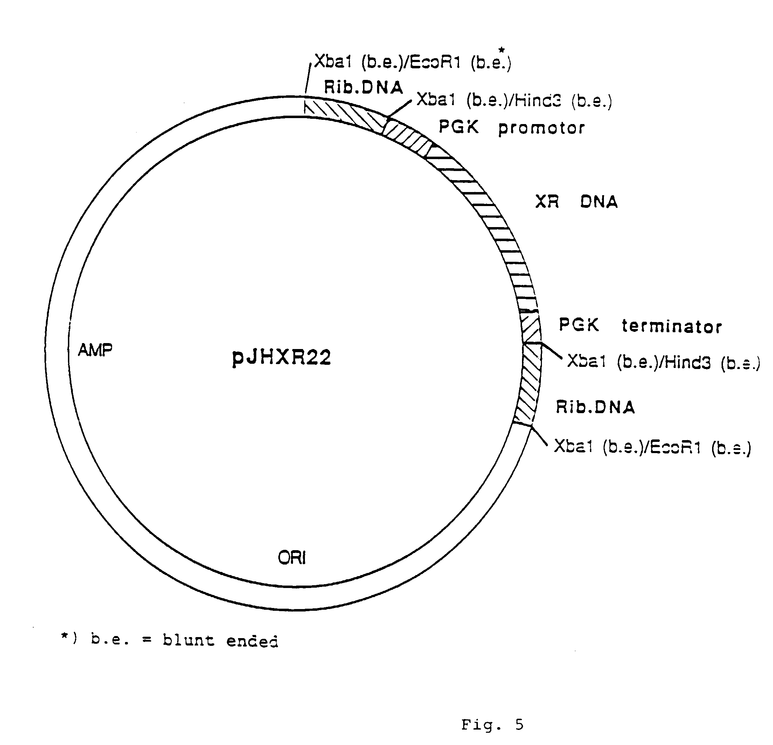

The gene coding for XR was also integrated into the yeast chromosome into the ribosomal RNA loci. The ribosomal sequences of plasmid pIRL9 were released with EcoRI, blunt-ended and cloned at the blunt-ended XbaI site of BS+ to obtain vector pJHR21. The gene coding for XR, coupled in between the *PGK* promoter and terminator was released from the vector pUA103 as a HindIII fragment, blunt-ended and cloned at the blunt-ended XbaI site in the ribosomal sequences of the plasmid pJHR21. From this resulting plasmid pJHXR22 (Fig. 5), the expression cassette, flanked by ribosomal sequences, was released by cutting in the unique restriction sites of the BS+ polylinker. This fragment was cotransformed into yeast with an autonomously replicating plasmid carrying a marker for transformation. The transformants obtained were screened for the presence of the gene coding for XR by enzyme activity tests as described above and the integration pattern was checked by Southern analysis. The autonomously replicating plasmid was removed from the cells carrying the XR expression cassette by cultivating the cells in non-selective YPD growth medium.

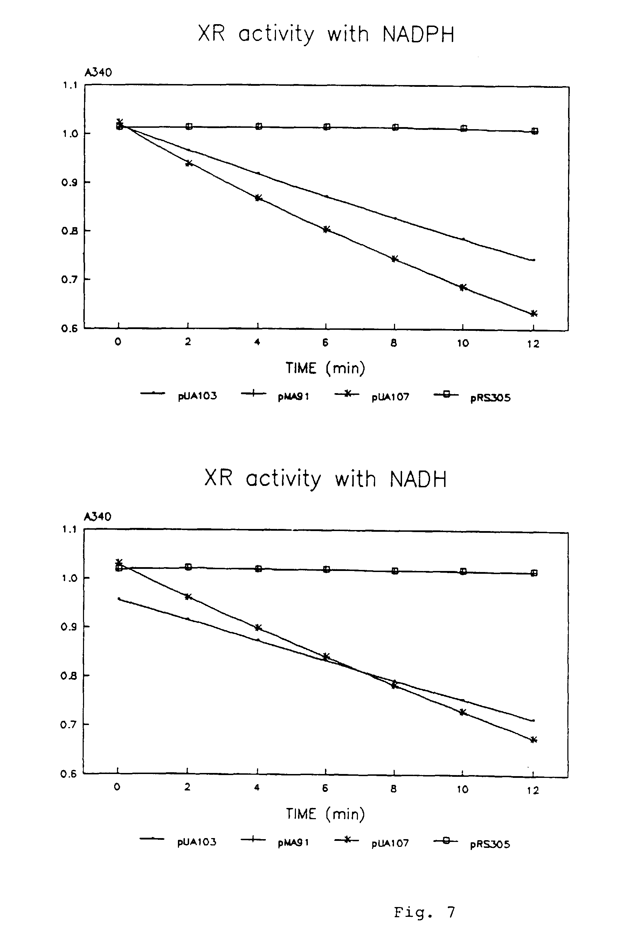

The transformants were grown in minimal selective medium and the expression of XR was analyzed by Western blotting using XR specific antibody and the alkaline phosphatase method of Promega (Fig. 6), and by enzyme activity measurements (Smiley and Bolen, 1982) from crude extracts of yeast cells broken by French press (Fig. 7).

### Preparative Example 6:

### Purification of XR from recombinant S. cerevisiae

A recombinant *S. cerevisiae* strain H477 was cultivated in a 1.5 l fermentor in a Sc-leu medium containing 20 g/l glucose. Growth was followed by turbidity measurements and the cells were collected at late exponential growth phase by centrifugation, washed and resuspended in 0.1 M phosphate buffer pH 7.0 containing 1.5 mM phenyl methyl sulfonyl fluoride, to a yeast concentration of 100 g dry weight/l. Cells were disrupted with 3 passes at 1000 bar through a high pressure homogenizer (French-press). The homogenate was partially clarified by 30 min centrifugation at 15 000 g. XR was purified from the clarified homogenate as described for *P. stipitis* in example 1.

### Example 7:

### Production of xylitol in vitro

A reaction mixture containing 0.33 M xylose, 0.33 M glucose-6-phosphate, 0.67 mM NADPH, 0.1 M phosphate buffer (pH 7.0), 1 nkat/ml XR activity of purified XR (Preparative Example 6) or from a diluted crude homogenate of the strain H475, and 1 nkat/ml glucose-6-phosphate dehydrogenase was incubated at 20°C for 5 h. Samples were withdrawn intermittently and analysed for xylitol using a xylitol kit from Boehringer. A constant xylitol production rate exceeding 0.14 g h⁻¹l⁻¹ was observed.

### Preparative Example 8:

### Co-expression of XR and XDH

The plasmids pUA103 and pUA107 were transformed into the strain VTT-C-91181 which carries the *xdh* gene on the plasmid pMW22. Transformants were selected, and also later kept, on Sc-leu-his-plates. The retainment of XDH activity was confirmed by a plate activity assay and the XR activity by enzyme activity measurement as described above. The two clones studied further, carrying the plasmids pUA103 and pMW22, were named H492 and H493.

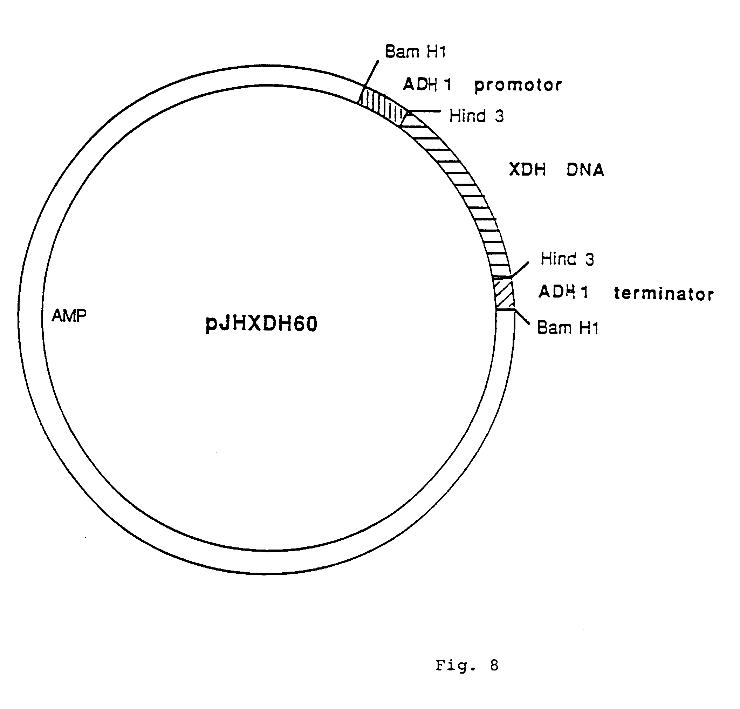

The full length *xdh* cDNA from the plasmid pJHXDH50 was cloned at the HindIII site in the vector pKB102 (Blomqvist *et al.* 1991) in between the yeast *ADH1* promoter and terminator. The expression cassette was released with BamHI from the resulting plasmid pJHXDH60 (Fig. 8) and cloned into the autonomously replicating yeast vector p3030 at the BamHI site generating the plasmid pJHXDH70. The resulting plasmid was transformed into the strain H477 carrying the gene encoding XR (Example 5) selecting the transformants on Sc-leu-his-plates. The expression of the *xdh* gene in *S. cerevisiae* was tested by enzyme activity measurement (Smiley and Bolen, 1982).

### Example 9:

### Production of xylitol in vivo by recombinant S. cerevisiae

The yeast strains H475 and H477 were cultivated in a 1.5 l fermentor in a medium containing 10 g/l yeast extract, 20 g/l bacto-peptone and 20 g/l glucose. Cultivation temperature was 30°C. pH was controlled between 4.5 and 8.0. Agitation speed was 400 rpm and aeration rate 0.5 vvm. When glucose was consumed according to the analysis of the samples from the broth, a feed of a solution containing 1 g glucose and 19 g xylose in 100 ml was started at a rate of 0.09 ml/min. After 83 hours of total cultivation time, xylitol concentration in the broth was 12.5 g/l as analysed by HPLC. Thus over 95 % yield of xylitol from xylose fed to the culture was achieved. By using the control strain carrying the vector pMA91 less than 8 % of the xylose was consumed in an analogous experiment.

### Example 10:

### Xylose fermentation by recombinant S. cerevisiae

*S. cerevisiae* strains H492 and H493 described in example 9 were cultivated on a rotary shaker in Sc-leu-his medium containing 20 g/l glucose. Rotating speed was 200 rpm. When both the glucose and ethanol formed were consumed, the broths were used as inoculum for fermentation on a rotary shaker in Sc-leu-his medium containing 20 g/l xylose. Rotating speed was 90 rpm. The consumption of xylose and the formation of ethanol and xylitol were followed during fermentation by taking samples and analysing them by HPLC (Hahn-Hägerdal *et al.,* 1986; Linden and Hahn-Hägerdal, 1989a, b).

### Example 11:

### Fermentation of xylose containing raw materials with recombinant S. cerevisiae

*S. cerevisiae* strains H492 and H493 were cultivated as in example 10 in a fermentation medium, where xylose was replaced by spent sulphite liquor. Xylose was converted to cells, ethanol and xylitol.

### References

Amore, R., Wilhelm, M. and Hollenberg, C.P. (1989) Appl. Microbiol. Biotechnol. 30: 351-357.

Baldari, C., Murray, J.A.H., Ghiara, P., Cesareni, G., and Galotti, C.L. (1987) EMBO J. 6: 229-234.

Barbosa, M.F.S., de Medeira, M.B., de Mancilha, I.M., Schneider, H. and Lee, H. 1988. Screening of yeasts for production of xylitol from D-xylose and some factors which affect xylitol yield in *Candida guilliermondii.* J. Ind. Microbiol. 3, 241-251.

Batt, C.A., Carvallo, S., Easson, D.D., Akedo, M. and Sinskey, A.J. 1986. Metabolic pathway in *Saccharomyces cerevisiae.* Biotechnology and Bioengineering 28, 549-553.

Bergmeyer, H.U., Gruber, W. and Gutmann, I. (1974) in Methods of Enzymatic Analysis (Bergmeyer, H.U., ed.) 2nd ed., vol. 3, pp. 1323-1330, Verlag Chemie, Weinheim and Academic Press, Inc., New York, London.

Blomqvist, K., Suihko, M.-L., Knowles, J. and Penttilä, M. Chromosomal integration and expression of two bacterial α-acetolactate decarboxylase genes in brewer's yeast. Submitted, 1991.

Bückmann, A.F. (1979) German Patent No 28.41.414.

Chiang, C. and Knight, S.G. 1960. Metabolism of D-xylose by moulds. Nature 188, No. 4744, 79-81.

Chirgwin, J.M., Przybyla, A.E., MacDonald, R.J. and Rutter, W.J. (1979) Isolation of biologically active ribonucleic acid from sources enriched in ribonuclease. Biochemistry 18: 5294-5299.

Clark, T.A. and Mackie, K.L. (1984) J. Chem. Tech. Biotechnol. 34b: 101-110.

Cryer, D.R., Eccleshall, R. and Marmur, J. (1975) Isolation of yeast DNA. Methods Cell Biol. 12: 39-44.

Gong, C.-S. (1985). US Pat. 4,511,656.

Güssow, D. and Clackson, T. (1989) Direct clone characterization from plaques and colonies by the polymerase chain reaction. Nucl. Acids Res. 17: 4000-.

Hahn-Hägerdal, B., Berner, S. and Skoog, K. 1986. Improved ethanol production from xylose with glucose isomerase and *Saccharomyces cerevisiae* using the respiratory inhibitor azide. Appl. Microbiol. Biotechnol. 24, 287-293.

Ito, H., Fukuda, Y., Murata, K. and Kimura, A. (1983) Transformation of intact yeast cells treated with alkali cations. J. Bact. 153: 163-168.

Kitpreechavanich, V., Nishio, N., Hayashi, M. and Nagai, S. 1985. Regeneration & retention of NADP(H) for xylitol production in an ionized membrane reactor. Biotechnol. Lett. 7, 657-662.

Kulbe, K.D., Schwab, U. and Gudernatsch, W. 1987. Enzyme-catalyzed production of mannitol and gluconic acid. Ann. N.Y. Acad. Sci. 506, 552-568.

Kulbe, K.D., Howaldt, M.W., Schmidt, K., Röthig, T.R. and Shmiel, H. 1989. Rejection and continuous regeneration of native coenzymes NAD(H)/NADP(H) in a charged ultrafiltration membrane enzyme reactor. Poster at the 10th Enzyme Engineering Conference, Sept. 24-29 1989, Kashikojima, Japan.

Ladisch, M.R., Lin, K.W., Voloch, M. and Tsao, G.T. (1983) Enzyme Microb. Technol. 5: 82-102.

Laemmli, U.K. (1970) Cleavage of structural proteins during the assembly of the head of bacteriophage T4. Nature 227: 680-685.

Lastick, S.M., Tucker, M.Y., Beyette, J.R., Noll, G.R. and Grohman, K. (1989) Appl. Microbiol. Biotechnol. 30: 574-579.

Lindén, T. and Hahn-Hägerdal, B. (1989a) Enzyme Microb. Technol. 11: 583-589.

Lindén, T. and Hahn-Hägerdal, B. (1989b) Biotechnol. Techniques 3: 189-192.

Maniatis, T., Fritsch, E.F. and Sambrook, J. (1982). Molecular cloning. A laboratory manual. Cold Spring Harbor Laboratory, New York.

Mellor, J., Dobson, M.J., Roberts, NA., Tuite, M.F., Emtage, J.S., White, S., Lowe, P.A., Patel, T., Kingsman, A.J. and Kingsman, S.M. (1983) Efficient synthesis of enzymatically active calf chymosin in *Saccharomyces cerevisiae.* Gene 24: 1-14.

Ojamo, H., Ylinen, L. and Linko, M. (1987) Finnish Patent 76377.

Onishi, H. and Suzuki, T. (1966) The production of xylitol, L-arabinitol and ribitol by yeasts. Agr. Biol. Chem. 30: 1139-1144.

Penttilä, M.E., Nevalainen, K.M.H., Raynal, A. and Knowles, J.K.C. 1984. Mol. Gen. Genet. 194: 494-499.

Prior, B.A., Kilian, S.G. and du Preez (1989) Process Biochemistry 2: 21-32.

Reslow, M., Adlercreutz, Mattiasson, B. (1988) Eur. J. Biochem. 177: 313-318.

Sarthy, A.V., McConaughy, B.L., Lobo, Z., Sundström, J.A., Furlong, C.E. and Hall, B.D. 1987. Expression of the *E. coli* xylose isomerase gene in *Saccharomyces cerevisiae.* Appl. Environ. Microbiol. 53, 1996-2000.

Senac, T. and Hahn-Hägerdal, B. (1990) Appl. Environ. Microbiol. (in press).

Sikorski, R.S. and Hieter, P. (1989) A system of shuttle vectors and yeast host strains designed for efficient manipulation of DNA in *Saccharomyces cerevisiae.* Genetics 122: 19-27.

Skoog, K. and Hahn-Hägerdal, B. (1988) Enzyme Microb. Technol. 10: 66-78.

Slininger, P.J., Bolen, O.L. and Kurtzman, C.P. (1987) Enzyme Microb. Technol. 9: 5-15.

Smiley, K.L. and Bolen, P.L. (1982) Demonstration of D-xylose reductase and D-xylitol dehydrogenase in *Pachysolen tannophilus.* Biotechnol. Lett. 4: 607.

Van Zyl, C., Prior, B.A., Kilian, S.G. and Kock, J.L.F. 1989. D-xylose utilization by *Saccharomyces cerevisiae.* J. Gen. Microbiol. 135, 2791-2798.

Wandrey, C., Wichmann, R., Leuchtenberger, W., Kula, M.-R. and Bückmann, A.F. (1981) US Patent No 4.304.858.

Wandrey, C., Wichmann, R., Leuchtenberger, W., Kula, M.-R. and Bückmann, A.F. (1982) US Patent No 4.326.031.

Yoshitake, J., Shimamura, M. and Imai, T. 1973a. Xylitol production by an *Enterobacter* species. Agr. Biol. Chem. 37, 2261-2267.

Yoshitake, J., Shimamura, M. and Imai, T., 1973b. Xylitol production by a *Corynebacterium* species. Agr. Biol. Chem. 37, 2251-2259.

Young, RA. and Davis, R.W. (1983) Yeast RNA polymerase II genes: isolation with antibody probes. Science 222: 778-782.

Yu, S., Wayman, M. and Parehk, S.R. (1987) Biotechnol. Bioeng. 29: 1144-1150.

Zagursky, R.J., Berman, M.L., Baumeister, K. and Lomax, N. (1986) Rapid and easy sequencing of large linear double stranded DNA and supercoiled plasmid DNA. Gene Anal. Techn. 2: 89-94.

### Deposited microorganism

The following yeast strain was deposited according to the Budapest Treaty at the National Collection of Yeast Cultures (NCYC), AFRC Institute of Food Research, Norwich Laboratory, Colney Lane, Norwich, NR4 7UA, UK

| Strain | Deposition number | Deposition date |
|---|---|---|
| *Saccharomyces cerevisiae* VTT-C-91181 carrying the plasmid pMW22 | NCYC 2352 | March 29, 1991 |

### Sequence Listing

### SEQ ID NO. 1

SEQUENCE TYPE: Peptide
SEQUENCE LENGTH: 9 amino acids
TOPOLOGY: linear
ORIGINAL SOURCE ORGANISM: *Pichia stipitis* CBS-6054
IMMEDIATE EXPERIMENTAL SOURCE: protein purification and N-terminal sequencing
FEATURES: from 1 to 9 amino acids of the mature protein
PROPERTIES: N-terminal peptide of xylose reductase (NADPH/NADH) enzyme

Pro Xaa Ile Lys Leu Asn Ser Gly Tyr

### SEQ ID NO. 2

SEQUENCE TYPE: Nucleotide with corresponding protein
SEQUENCE LENGTH: 954 base pairs
STRANDEDNESS: single
TOPOLOGY: linear
MOLECULE TYPE: cDNA to mRNA, genomic DNA
ORIGINAL SOURCE ORGANISM: *Pichia stipitis* CBS-6054
IMMEDIATE EXPERIMENTAL SOURCE: cDNA library, PCR product from genomic DNA
FEATURES: from 1 to 954 bp mature protein
PROPERTIES: xylose reductase (NADPH/NADH) activity of the product

ATG CCT TCT ATT AAG TTG AAC TCT GGT TAC GAC ATG CCA GCC GTC 45
Met Pro Ser Ile Lys Leu Asn Ser Gly Tyr Asp Met Pro Ala Val
1               5                   10                  15

GGT TTC GGC TGT TGG AAA GTC GAC GTC GAC ACC TGT TCT GAA CAG 90
Gly Phe Gly Cys Trp Lys Val Asp Val Asp Thr Cys Ser Glu Gln
                20                  25                  30

ATC TAC CGT GCT ATC AAG ACC GGT TAC AGA TTG TTC GAC GGT GCC 135
Ile Tyr Arg Ala Ile Lys Thr Gly Tyr Arg Leu Phe Asp Gly Ala
                35                  40                  45

GAA GAT TAC GCC AAC GAA AAG TTA GTT GGT GCC GGT GTC AAG AAG 180
Glu Asp Tyr Ala Asn Glu Lys Leu Val Gly Ala Gly Val Lys Lys
                50                  55                  60

GCC ATT GAC GAA GGT ATC GTC AAG CGT GAA GAC TTG TTC CTT ACC 225
Ala Ile Asp Glu Gly Ile Val Lys Arg Glu Asp Leu Phe Leu Thr
                65                  70                  75

TCC AAG TTG TGG AAC AAC TAC CAC CAC CCA GAC AAC GTC GAA AAG 270
Ser Lys Leu Trp Asn Asn Tyr His His Pro Asp Asn Val Glu Lys
                80                  85                  90

GCC TTG AAC AGA ACC CTT TCT GAC TTG CAA GTT GAC TAC GTT GAC 315
Ala Leu Asn Arg Thr Leu Ser Asp Leu Gln Val Asp Tyr Val Asp
                95                  100                 105

TTG TTC TTG ATC CAC TTC CCA GTC ACC TTC AAG TTC GTT CCA TTA 360
Leu Phe Leu Ile His Phe Pro Val Thr Phe Lys Phe Val Pro Leu
                110                 115                 120

GAA GAA AAG TAC CCA CCA GGA TTC TAC TGT GGT AAG GGT GAC AAC 405
Glu Glu Lys Tyr Pro Pro Gly Phe Tyr Cys Gly Lys Gly Asp Asn
                125                 130                 135

TTC GAC TAC GAA GAT GTT CCA ATT TTA GAG ACC TGG AAG GCT CTT 450
Phe Asp Tyr Glu Asp Val Pro Ile Leu Glu Thr Trp Lys Ala Leu
                140                 145                 150

GAA AAG TTG GTC AAG GCC GGT AAG ATC AGA TCT ATC GGT GTT TCT 495
Glu Lys Leu Val Lys Ala Gly Lys Ile Arg Ser Ile Gly Val Ser
                155                 160                 165

AAC TTC CCA GGT GCT TTG CTC TTG GAC TTG TTG AGA GGT GCT ACC 540
Asn Phe Pro Gly Ala Leu Leu Leu Asp Leu Leu Arg Gly Ala Thr
                170                 175                 180

ATC AAG CCA TCT GTC TTG CAA GTT GAA CAC CAC CCA TAC TTG CAA 585
Ile Lys Pro Ser Val Leu Gln Val Glu His His Pro Tyr Leu Gln
                185                 190                 195

CAA CCA AGA TTG ATC GAA TTC GCT CAA TCC CGT GGT ATT GCT GTC 630
Gln Pro Arg Leu Ile Glu Phe Ala Gln Ser Arg Gly Ile Ala Val
                200                 205                 210

ACC GCT TAC TCT TCG TTC GGT CCT CAA TCT TTC GTT GAA TTG AAC 675
Thr Ala Tyr Ser Ser Phe Gly Pro Gln Ser Phe Val Glu Leu Asn
                215                 220                 225

CAA GGT AGA GCT TTG AAC ACT TCT CCA TTG TTC GAG AAC GAA ACT 720
Gln Gly Arg Ala Leu Asn Thr Ser Pro Leu Phe Glu Asn Glu Thr
                230                 235                 240

ATC AAG GCT ATC GCT GCT AAG CAC GGT AAG TCT CCA GCT CAA GTC 765
Ile Lys Ala Ile Ala Ala Lys His Gly Lys Ser Pro Ala Gln Val
                245                 250                 255

TTG TTG AGA TGG TCT TCC CAA AGA GGC ATT GCC ATC ATT CCA AAG 810
Leu Leu Arg Trp Ser Ser Gln Arg Gly Ile Ala Ile Ile Pro Lys
                260                 265                 270

TCC AAC ACT GTC CCA AGA TTG TTG GAA AAC AAG GAC GTC AAC AGC 855
Ser Asn Thr Val Pro Arg Leu Leu Glu Asn Lys Asp Val Asn Ser
                275                 280                 285

TTC GAC TTG GAC GAA CAA GAT TTC GCT GAC ATT GCC AAG TTG GAC 900
Phe Asp Leu Asp Glu Gln Asp Phe Ala Asp Ile Ala Lys Leu Asp
                290                 295                 300

ATC AAC TTG AGA TTC AAC GAC CCA TGG GAC TGG GAC AAG ATT CCT 945
Ile Asn Leu Arg Phe Asn Asp Pro Trp Asp Trp Asp Lys Ile Pro
                305                 310                 315

ATC TTC GTC 954
Ile Phe Val

## Claims

1. A process for producing xylitol, which process comprises
a) cultivating in a xylose containing medium a yeast strain transformed with a DNA sequence encoding a xylose reductase enzyme, which DNA sequence when transformed into said yeast strain confers to said yeast strain the ability of reducing xylose to xylitol, and
b) recovering xylitol formed from the medium.

2. The process of claim 1, wherein said DNA sequence encodes a polypeptide comprising essentially the amino acid sequence of SEQ. ID. NO. 2, or a fragment thereof wherein said polypeptide or fragment displays xylose reductase activity.

3. The process of claim 1 or 2, wherein said transformed yeast strain belongs to the species *Saccharomyces cerevisiae, Kluyveromyces spp., Schizosaccharomyces pombe* or *Pichia spp..*

4. The process of any one of claims 1 to 3, wherein said transformed yeast strain belongs to the species *Saccharomyces cerevisiae.*

5. The process of any one of claims 1 to 4, wherein said transformed yeast strain is *Saccharomyces cerevisiae* H475 or H477, obtainable by transforming *Saccharomyces cerevisiae* GPY55-15Bα and S150-2B respectively with plasmid pUA103 as shown in Figure 1.

6. The process of claim 1, wherein at least one additional carbon source is present in the medium to enhance regeneration of a cofactor selected from NADPH and NADH required for xylose reductase activity.

7. The process of claim 6, wherein said additional carbon source is ethanol, glucose or glycerol.

8. The process of claim 1, wherein said transformed yeast strain is further transformed with a second DNA sequence which directs expression in said yeast strain of a xylitol dehydrogenase enzyme, said xylitol dehydrogenase enzyme making possible the regeneration of a cofactor required for xylose reductase activity.

9. The process of claim 8, wherein said transformed yeast strain belongs to the species *Saccharomyces cerevisiae, Kluyveromyces spp., Schizosaccharomyces pombe* or *Pichia spp.*

10. The process of claim 9 wherein said transformed yeast strain is *Saccharomyces cerevisiae* VTT-C-91181 carrying the plasmid pMW22 (NCYC no. 2352) additionally transformed with plasmid pUA103 as shown in Figure 1. .

11. An enzymatic process for producing xylitol, which process comprises
a) cultivating a recombinant yeast strain transformed with a DNA sequence encoding a xylose reductase enzyme under conditions permitting expression of said xylose reductase,
b) recovering said xylose reductase enzyme,
c) using said xylose reductase in an enzyme reactor with a cofactor regenerating system, and
d) isolating and purifying the xylitol produced.

## Patentansprüche

1. Verfahren zur Produktion von Xylit, umfassend
a) Züchtung eines Hefestammes, der mit einer ein Xylose-Reduktaseenzym codierenden DNA-Sequenz transformiert ist, in einem Xylose enthaltenden Medium, wobei die DNA-Sequenz, wenn sie in den Hefestamm transformiert ist, dem Hefestamm die Fähigkeit verleiht, Xylose zu Xylit zu reduzieren, und
b) Gewinnung des gebildeten Xylits aus dem Medium.

2. Verfahren nach Anspruch 1, wobei die DNA-Sequenz ein Polypeptid codiert, das im wesentlichen die Aminosäuresequenz von Sequenz ID No. 2 umfaßt, oder ein Fragment davon, wobei das Polypeptid oder Fragment Xylose-Reduktaseaktivität aufweist.

3. Verfahren nach Anspruch 1 oder 2, wobei der transformierte Hefestamm zu der Art *Saccharomyces cerevisiae, Kluyveromyces* spp., *Schizosaccharomyces pombe* oder *Pichia* spp. gehört.

4. Verfahren nach einem der Ansprüche 1 bis 3, wobei der transformierte Hefestamm zur Art *Saccharomyces cerevisiae* gehört.

5. Verfahren nach einem der Ansprüche 1 bis 4, wobei der transformierte Hefestamm *Saccharomyces cerevisiae* H475 oder H477 ist, erhältlich durch Transformation von *Saccharomyces cerevisiae* GPY55-15Bα bzw. S150-2B mit dem Plasmid pUA103, wie in Fig. 1 gezeigt.

6. Verfahren nach Anspruch 1, wobei mindestens eine zusätzliche Kohlenstoffquelle im Medium vorliegt, so daß die Regeneration eines Cofaktors verstärkt wird, ausgewählt aus NADPH und NADH, die für die Xylose-Reduktase-Aktivität erforderlich sind.

7. Verfahren nach Anspruch 6, wobei die zusätzliche Kohlenstoffquelle Ethanol, Glucose oder Glycerin ist.

8. Verfahren nach Anspruch 1, wobei der transformierte Hefestamm zusätzlich mit einer zweiten DNA-Sequenz transformiert ist, die die Expression eines Xylit-Dehydrogenaseenzyms in dem Hefestamm bewirkt, wobei das Xylit-Dehydrogenaseenzym die Regeneration eines Cofaktors möglich macht, der für Xylose-Reduktase-Aktivität erforderlich ist.

9. Verfahren nach Anspruch 8, wobei der transformierte Hefestamm zur Art *Saccharomyces cerevisiae, Kluyveromyces* spp., *Schizosaccharomyces pombe* oder *Pichia* spp. gehört.

10. Verfahren nach Anspruch 9, wobei der transformierte Hefestamm der das Plasmid pMW22 (NCYC Nr. 2352) tragende *Saccharomyces cerevisiae*-Stamm VTT-C-91181 ist, der zusätzlich transformiert ist mit Plasmid pUA103 wie in Fig. 1 gezeigt.

11. Enzymatisches Verfahren zur Produktion von Xylit, umfassend
a) Züchtung eines rekombinanten Hefestammes, der mit einer ein Xylose-Reduktaseenzym codierenden DNA-Sequenz transformiert ist, unter Bedingungen, die die Expression der Xylosereduktase ermöglichen,
b) Gewinnung des Xylose-Reduktaseenzyms,
c) Verwendung der Xylose-Reduktase in einem Enzymreaktor mit einem Cofaktor-regenerierenden System, und
d) Isolierung und Reinigung des produzierten Xylits.

## Revendications

1. Procédé de production de xylitol, lequel procédé consiste :
a) à cultiver dans un milieu contenant du xylose une souche de levure transformée à l'aide d'une séquence d'ADN codant une enzyme xyloseréductase, laquelle séquence d'ADN, après transformation dans ladite souche de levure, confère à ladite souche de levure le pouvoir de réduire le xylose en xylitol, et
b) à récupérer du milieu le xylitol formé.

2. Procédé selon la revendication 1, dans lequel ladite séquence d'ADN code un polypeptide comprenant essentiellement la séquence d'acides aminés SEQ. ID. NO. 2 ou l'un de ses fragments, où ledit polypeptide ou fragment présente une activité de xyloseréductase.

3. Procédé selon la revendication 1 ou 2, dans lequel ladite souche de levure transformé appartient aux espèces *Saccharomyces cerevisiae, Kluyveromyces spp., Schizosaccharomyces pombe* ou *Pichia spp.*

4. Procédé selon l'une quelconque des revendications 1 à 3, dans lequel ladite souche de levure transformée appartient à l'espèce *Saccharomyces cerevisiae.*

5. Procédé selon l'une quelconque des revendications 1 à 4, dans lequel ladite souche de levure transformée est *Saccharomyces cerevisiae* H475 ou H477, qui peut être obtenue par transformation, avec le plasmide pUA103 tel que présenté sur la Figure 1, de *Saccharomyces cerevisiae,* respectivement GPY55-15Bα et S150-2B.

6. Procédé selon la revendication 1, dans lequel au moins une source de carbone additionnel est présente dans le milieu pour renforcer la régénération d'un cofacteur sélectionné parmi NADPH et NADH, nécessaire à l'activité de xyloseréductase.

7. Procédé selon la revendication 6, dans lequel ladite source de carbone additionnel est l'éthanol, le glucose ou le glycérol.

8. Procédé selon la revendication 1, dans lequel ladite souche de levure transformée est soumise à une transformation plus poussée avec une deuxième séquence d'ADN qui dirige l'expression dans ladite souche de levure d'une enzyme xylitol-déshydrogénase, ladite enzyme xylitol-déshydrogénase permettant la régénération d'un cofacteur nécessaire à l'activité de xyloseréductase.

9. Procédé selon la revendication 8, dans lequel ladite souche de levure transformée appartient à l'espèce *Saccharomyces cerevisiae, Kluyveromyces spp.*, *Schizosaccharomyces pombe* ou *Pichia spp.*

10. Procédé selon la revendication 9, dans lequel ladite souche de levure transformée est *Saccharomyces cerevisiae* VTT-C-91181, qui porte le plasmide pMW22 (NCYC N° 2352) ayant subi une transformation supplémentaire par le plasmide pUA103 comme on le voit sur la Figure 1.

11. Procédé enzymatique pour produire le xylitol, lequel procédé consiste :
a) à cultiver une souche de levure recombinante transformée avec une séquence d'ADN codant une enzyme xyloseréductase dans des conditions permettant l'expression de ladite xyloseréductase,
b) à récupérer ladite enzyme xyloseréductase,
c) à utiliser ladite xyloseréductase dans un réacteur enzymatique avec un système de régénération d'un cofacteur, et
d) à isoler et purifier le xylitol produit.
